# EUROPEAN PATENT APPLICATION

(11) **EP 2 316 445 A1**
(43) Date of publication of application: **04.05.2011**
(21) Application number: 09762524.8
(22) Date of filing: 11.06.2009
(51) Int. Cl.: A61K 31/202, A61K 31/20, A61K 31/201, A61P 3/04, A61P 3/06, A61P 3/10, A61P 9/12, A61P 19/06, A61P 43/00

(54) **HUMAN beta3 ADRENERGIC RECEPTOR LIGAND, AND FOOD OR PHARMACEUTICAL PRODUCT CONTAINING THE SAME**

(30) Priority: 11.06.2008 JP 2008153390
(71) Applicant: Ricom Corporation, Tokyo 171-0022 (JP)
(72) Inventor: FUJIMOTO Yasuo, Tokyo 178-0061 (JP); KURIHARA Shoichi, Tokyo 135-0016 (JP); HAMAYA Tadao, Tokyo 112-0002 (JP)
(74) Representative: HOFFMANN EITLE
(86) International application number: PCT/JP2009/060682
(87) International publication number: WO 2009/151094

(57) **Abstract**

A human adrenergic ß₃ receptor ligand containing the following 3 components: (A) an unsaturated fatty acid having not less than three double bonds or a pharmaceutically acceptable salt thereof; (B) an unsaturated fatty acid having one or two double bond(s) or a pharmaceutically acceptable salt thereof; and (C) a saturated fatty acid or a pharmaceutically acceptable salt thereof; and a food product and a pharmaceutical containing it, especially, an agent for prophylaxis and/or amelioration of lifestyle-related diseases such as obesity, adipositas, diabetes, hyperlipemia, hypertension and/or gout.

## Description

### TECHNICAL FIELD

The present invention relates to a human ß₃ adrenergic receptor ligand, and a food product and a pharmaceutical containing it.

### BACKGROUND ART

Adrenergic receptors are receptors which bind to catecholamine agonists such as adrenaline and noradrenaline released from sympathetic nerves, and can be divided into two groups, α receptors and β receptors. Adrenergic α receptors show higher sensitivities to agonists in the order of noradrenaline ≥ adrenaline > dopamine > isoproterenol, and adrenergic β receptors show higher sensitivities to agonists in the order of isoproterenol > adrenaline ≥ noradrenaline > dopamine.

Adrenergic β receptors include ß₁, ß₂ and ß₃ receptors, and the existence of ß₄ receptors has been suggested recently. In terms of the actions of ligands for the respective receptors, it is known that adrenergic ß₁ receptor agonists have heart rate increasing actions; adrenergic ß₁ receptor antagonists have antihypertensive actions; adrenergic ß₂ receptor agonists have bronchial smooth muscle-relaxing actions; and adrenergic ß₃ receptor agonists have thermogenesis-activating actions and lipolysis-promoting actions. Thus, there is a concern that agents which activate sympathetic nerves to promote secretion of catecholamine agonists, and nonselective adrenergic β receptor agonists may cause side effects due to the actions of ß₁ and ß₂, so that these are not suitable for prophylaxis and/or amelioration of lifestyle-related diseases such as obesity. Therefore, for prophylaxis and/or amelioration of lifestyle-related diseases such as obesity, adrenergic ß₃ receptor agonists are effective.

The agonists as adrenergic ß₃ receptor ligands were first discovered in 1984, and their anti-obesity actions and antidiabetic actions due to thermogenesis and lipolysis were confirmed in animal experiments. However, these actions were weak in human. The cause of such a difference in the actions was revealed in 1989 to be the specific difference in the chemical structure of adrenergic ß₃ receptors between rodents such as mice and rats, and human (Non-patent Documents 1 and 2). Therefore, human adrenergic ß₃ receptor ligands, especially, agonists, are effective for prophylaxis and/or amelioration of lifestyle-related diseases such as obesity and diabetes, and their development is demanded.

Recently, several compounds have been known as human adrenergic ß₃ receptor agonists (Non-patent Document 1), and their actions as anti-obesity drugs and antidiabetic drugs have been confirmed in clinical tests. Further, a human ß₃ adrenergic receptor agonist agent containing as an effective component an extract from Japanese pepper has been reported (Patent Document 1).

On the other hand, it has been reported that chitosan-containing polysaccharides produced from mushrooms such as *Agaricus, Lentinus edodes* (shiitake mushroom), *Flamm ulina velutipes* (enokitake mushroom), *Lyophyllum* (shimeji mushroom), *Grifola frondosa* (maltake mushroom) and *Pholiota nameko* (nameko mushroom) have actions to cause reduction in the blood pressure, urine sugar level, blood glucose level, uric acid level, total cholesterol level, neutral fat level and the like, and therefore are effective for improvement of the values obtained by tests for lifestyle-related diseases such as hypertension and obesity (Patent Document 2).

[Patent Document 1] JP 2006-96666 A
[Patent Document 2] WO 2004/033502
[Non-patent Document 1] Yasuto Takakura, Toshihide Yoshida, Folia Pharmacol. Jpn., 118, 315-320, (2001)
[Non-patent Document 2] C. Weyer, et al., Diabetes & Metabolism, 25, 11-21, (1999)

### DISCLOSURE OF THE INVENTION

### PROBLEMS TO BE SOLVED BY THE INVENTION

The present invention aims to provide a human adrenergic ß₃ receptor ligand.
Another object of the present invention is to provide a food product and a pharmaceutical containing the above-described human adrenergic ß₃ receptor ligand.
Still another object of the present invention is to provide an agent for prophylaxis and/or amelioration of lifestyle-related diseases such as obesity, adipositas, diabetes, hyperlipemia, hypertension and gout, comprising a human adrenergic ß₃ receptor ligand.

### MEANS FOR SOLVING THE PROBLEMS

After various considerations to achieve the above objects, the present inventors discovered that particular combinations of unsaturated fatty acids and a saturated fatty acid have high binding activities to human adrenergic ß₃ receptors, thereby completing the present invention.
The present invention provides a human adrenergic ß₃ receptor ligand comprising as effective components the following particular combination of unsaturated fatty acids and a saturated fatty acid, and a food product and a pharmaceutical containing it.

1. A human adrenergic ß₃ receptor ligand comprising the three components described below:
   (A) an unsaturated fatty acid having not less than three double bonds or a pharmaceutically acceptable salt thereof;
   (B) an unsaturated fatty acid having one or two double bond(s) or a pharmaceutically acceptable salt thereof; and
   (C) a saturated fatty acid or a pharmaceutically acceptable salt thereof.
2. The human adrenergic ß₃ receptor ligand according to 1 above, wherein the number of carbon atoms in the unsaturated fatty acid of the component (A) is 8 to 24.
3. The human adrenergic ß₃ receptor ligand according to 1 or 2 above, wherein the number of carbon atoms in the unsaturated fatty acid of the component (B) is 8 to 24.
4. The human adrenergic ß₃ receptor ligand according to any one of 1 to 3 above, wherein the number of carbon atoms in the saturated fatty acid of the component (C) is 8 to 24.
5. The human adrenergic ß₃ receptor ligand according to any one of 1 to 4 above, wherein the mass ratio between the component (A) and the component (B) is 1:99 to 8:2.
6. The human adrenergic ß₃ receptor ligand according to any one of 1 to 5 above, wherein the ratio between the total mass of the component (A) and the component (B) and the mass of the component (C) is 50:1 to 1:3.
7. A food product containing the human adrenergic ß₃ receptor ligand according to any one of 1 to 6 above.
8. A pharmaceutical containing the human adrenergic ß₃ receptor ligand according to any one of 1 to 6 above.

### EFFECT OF THE INVENTION

The human adrenergic ß₃ receptor ligand of the present invention shows a high binding activity to human adrenergic ß₃ receptors and has actions to cause reduction in the blood pressure, urine sugar level, blood glucose level, uric acid level, total cholesterol level, neutral fat level, visceral fat level and the like, and is effective for prophylaxis and/or therapy of lifestyle-related diseases such as hypertension, diabetes, obesity, hypercholesterolemia and hyperlipemia. Further, since the toxicity of the effective component of the present invention is low, it can be safely used for food products and pharmaceuticals.

### BEST MODE FOR CARRYING OUT THE INVENTION

The carbon number of unsaturated fatty acid of the effective component (A) having not less than three double bonds is preferably 8 to 24, and more preferably 10 to 20.
Examples of the triunsaturated fatty acid include α-linolenic acid (18:3, 9,12,15-triunsaturated fatty acid), α-eleostearic acid (18:3, 9c,11t,13t), ß-eleostearic acid (18:3, 9t,11t,13t), punicic acid (18:3, 9c,11t,13c), calendic acid (18:3, 8t, 10t, 12c), jarcaric acid (18:3, 8c, 10t, 12c), catalpic acid (18:3, 9t,11t,13c) and kamlolenic acid (180H, 9c, 11t, 13t); examples of the tetraunsaturated fatty acid include stearidonic acid (6, 9, 12, 15-tetraunsaturated fatty acid), arachidonic acid (5,8,11,14-tetraunsaturated fatty acid) and parinaric acid (18:4, 9c, 11t,13t,15c); examples of the pentaunsaturated fatty acid include eicosapentaenoic acid (all-cis-icosa-5,8,11,14,17-pentaenoic acid) and clupanodonic acid (7,10,13,16,19-pentaunsaturated fatty acid); and examples of the hexaunsaturated fatty acid include docosahexaenoic acid.
Examples of especially preferred unsaturated fatty acids include α-linolenic acid, eicosapentaenoic acid and docosahexaenoic acid.

The unsaturated fatty acid of the effective component (B) having one or two double bond(s) desirably has a carbon number of preferably 8 to 24, more preferably 10 to 20.
Examples of the monounsaturated fatty acid include myristoleic acid (carbon number: 14), palmitoleic acid (carbon number 16), oleic acid (carbon number 18), elaidic acid (carbon number 18), vaccenic acid (carbon number 18), gadoleic acid (carbon number 20), erucic acid (carbon number 22) and nervonic acid (carbon number 24); and examples of the diunsaturated fatty acid include linoleic acid (carbon number 18).

The saturated fatty acid of the effective component (C) of the present invention desirably has a carbon number of preferably 8 to 24, more preferably 10 to 20.
Particular examples thereof include octanoic acid, nonanoic acid, decanoic acid, dodecanoic acid, tetradodecanoic acid, pentadecanoic acid, hexadecanoic acid, heptadecanoic acid, octadecanoic acid, nonadecanoic acid, icosanoic acid, docosanoic acid, tetradocosanoic acid, hexadocosanoic acid, octadocosanoic acid and triacontanoic acid.

In the present invention, the effective components (A), (B) and (C) may be in the forms of salts, esters and/or amides.
The salts are not restricted as long as they are sitologically, nutritionally or pharmaceutically acceptable, and examples thereof include metal salts such as sodium salts and calcium salts; ammonium salts; salts with organic bases such as methylamine, ethylamine, diethylamine, triethylamine, pyrrolidine, piperidine, morpholine, hexamethyleneimine, aniline and pyridine; and salts with amino acids such as arginine, glutamic acid and ornithine.
Further, any ester derivatives can be selected as long as they are sitologically, nutritionally or pharmaceutically acceptable, and ethyl esters, butyl esters, propyl esters and glycerol esters are preferred; and ethyl esters and glycerol esters are more preferred. The glycerol derivatives may be in the form of any of monoglycerides, diglycerides and triglycerides, wherein the forms of diglycerides and triglycerides are preferred, and triglycerides are most preferred.
Further, the amide derivatives are not restricted as long as they are sitologically, nutritionally or pharmaceutically acceptable, and examples thereof include acetamide, propionamide, butylamide and valeramide.

In the present invention, the mass ratio between the component (A) and the component (B) is preferably 1:90 to 8:2, more preferably 1:60 to 3:7.
Further, the ratio between the total mass of the component (A) and the component (B) and the mass of the component (C) is preferably 50:1 to 1:3, more preferably 40:1 to 1:1.

The adrenergic ß3 receptor ligand of the present invention can be used solely as an effective component, but can also be used in a form wherein a vehicle and/or the like is/are added.
For example, in order to use the ligand of the present invention in the form of a solution, a preservative such as sodium benzoate, methyl p-oxybenzoate or sodium dehydroacetate; a solubilizer such as malic acid, ascorbic acid, citric acid or acetic acid; and/or a coloring agent, perfume, flavoring agent and/or a sweetener such as glucose or mannitol is/are blended as required in addition to the above effective component, and further, a diluent such as distilled water or physiological saline is added as required, to prepare a pharmaceutical or a food product.

The pharmaceutical containing the above component as an effective component is usually prepared into the form of a solid preparation such as a tablet, pill, powder, granule, capsule or suppository. Such a pharmaceutical preparation is prepared using a diluting agent or a vehicle, such as a filler, bulking agent, binder, wetting agent, disintegrator, surfactant and/or lubricant, which is/are normally employed.
When the pharmaceutical is formed into a tablet, carriers which are conventionally known in the art can be widely used, and examples thereof include vehicles such as lactose, mannitol, saccharose, sodium chloride, glucose, starch, calcium carbonate, kaolin and crystalline cellulose; binders such as distilled water, physiological saline, simple syrup, glucose solution, starch solution, gelatin solution, carboxymethyl cellulose, potassium phosphate and polyvinyl pyrrolidone; disintegrators such as dry starch, sodium alginate, powdered agar, sodium hydrogen carbonate, calcium carbonate, sodium lauryl sulfate, monoglyceride stearate, starch and lactose; disintegration suppressing agents such as saccharose, stearin, cacao butter and hydrogenated oil; dissolution/absorption enhancers such as acetic acid, ascorbic acid and malic acid; adsorbing agents such as glycerin, starch, lactose, kaolin, bentonite and colloidal silicic acid; and lubricants such as purified talc, stearate and polyethylene glycol. Further, the tablet may be made, as required, into a sugar-coated tablet, gelatin-coated tablet, enteric-coated tablet, film-coated tablet, or double-layer tablet or multilayer tablet.

For forming the pharmaceutical into a pill, carriers conventionally known in the art can be widely used, and examples thereof include vehicles such as glucose, lactose, mannitol, starch, cacao butter, hydrogenated vegetable oil, kaolin and talc; and disintegrators such as gum arabic powder and gelatin. For forming the pharmaceutical into a suppository, carriers conventionally known in the art can be widely used, and examples thereof include cacao butter, esters of higher alcohols, and gelatin.
The content of the effective component is not restricted and can be widely selected, and the total content of the effective components (A), (B) and (C) contained in the preparation may be usually 0.001 to 30% by mass, preferably 0.01 to 10% by mass.
The dose is not restricted, and may be appropriately selected depending on the conditions such as the dose regimen, age and sex of the patient, and the severity of the disease. For example, a total of 0.01 to 20 mg, preferably 0.02 to 10 mg of the effective components (A), (B) and (C) per 1 kg of the body weight is dividedly orally administered in 1 to 4 times per day.
The food product containing the effective components (A), (B) and (C) of the present invention is not restricted, and examples thereof include soups, miso soups, drinks, jellies and gummies. The total content of the effective components (A), (B) and (C) is preferably 0.001 to 30% by mass, more preferably 0.01 to 10% by mass.

The adrenergic ß₃ receptor binding activity can be measured according to, for example, the method described in Cell Biology: Feve et al, Proc. Natl. Acad. Sci. USA 91(1994), Vol.91, pp.5677-5681, using HEK-293 cells which express a human recombinant adrenergic ß₃ receptor. That is, a solution of a sample in 1% DMSO, and 0.5 nM [¹²⁵I]cyanopindolol are added to Tris buffer (pH 7.4), and the HEK-293 cells are cultured therein at 25°C for 90 minutes, followed by filtration and washing of the cells, and measurement of the radioactivity of the adrenergic ß₃ receptor binding ligand.

### EXAMPLES

The present invention will now be described in detail by way of Examples below.

### Test Example 1

Using 4 types of rat primary preadipocytes (visceral fat, periepididymal fat, subcutaneous white fat and brown fat), the fat accumulation-suppressing effect and fat-releasing effect by the composition of the present invention were preliminarily studied in vitro.

### Test Material and Test Method

### Negative Control Substance

Dimethyl Sulfoxide (hereinafter referred to as DMSO)(Wako Pure Chemical Industries, Ltd.) was used.

### Test Substance

Each component containing the components shown in Table 1 below was dissolved in DMSO to 1 w/v%, and the resulting solution was added to an adipocyte differentiation medium (Primary Cell Co., Ltd., Lot No. 080411) to a final concentration of 10 µg/mL before use. The test of the adrenergic ß₃ receptor binding activity was carried out by the above-mentioned method.

**[Table 1]**

| Compound | Example | | | | Comparative Example | | | | | | | | |
|---|---|---|---|---|---|---|---|---|---|---|---|---|---|
| | 1 | 2 | 3 | 4 | 1 | 2 | 3 | 4 | 5 | 6 | 7 | 8 | 9 |
| | | | | | | | | | | | | | |
| C12:0 | | | | 1 | | | | | | | | | |
| C15:0 | 1 | 1 | | | | 5 | | | | | | | |
| C16:0 | 1 | 0.5 | 9 | | | | 5 | 1 | | 10 | | | |
| C17:0 | | | | | | | | | 1 | | | | |
| C18:0 | | | | | | | | | | | | 5 | 1 |
| C18:1 | 1 | | 3 | | | | | | | | | | |
| C18:2 | 1 | 44 | 2 | 1 | 10 | 1 | 1 | 10 | 10 | | | | |
| C18:3α | 3 | 1 | 1 | 1 | | | | | | 1 | 10 | 1 | 5 |
| | | | | | | | | | | | | | |
| Binding activity (%) | 71 | 67 | 72 | 56 | 34 | 14 | 35 | -8 | -13 | -15 | 22 | -1 | 43 |

### Cells Employed

The Preadipocyte Culture kit H-3 with 3 types of cells (primary, rat, Primary Cell Co., Ltd.: visceral preadipocytes (hereinafter referred to as VAC), Lot No. FIHA-FV; subcutaneous white preadipocytes (hereinafter referred to as SAC), FIHA-FS; periepididymal preadipocytes (hereinafter referred to as EAC), FIHA-FE) and the Brown Adipocyte Culture kit F-8 (primary, rat, Primary Cell Co., Ltd.: Lot No. HDOA-1) were used.
Norepinephrine (hereinafter referred to as NE) (-)-Norepinephrine(+)-bitartrate salt hydrate (SIGMA: Lot No. 103K0979) was used.

### Preparation of Cell Lysate

TRI-Reagent (Molecular Research Center: Lot No. 3681) was used.

### Test Method

### Operation of Culture

### (1) Three Types of Preadipocytes (VAC, SAC and EAC)

The 3 types of preadipocytes (visceral fat, periepididymal fat and subcutaneous white fat) were seeded into 12 wells in a 24-well plate at a cell number of 1.5×10⁶ (1.2×10⁵ cells/mL/well), followed by preculture for 4 days in the adipocyte differentiation medium. Thereafter (on day 4 after seeding), the negative control substance and the test substance were prepared to a predetermined concentration in a visceral fat differentiation medium, and the prepared medium was added to the cells.
The medium was replaced on days 0, 2 and 4 after the addition of the test substance (days 4, 6 and 8 after seeding), while adding freshly prepared medium after the collection. On the last day, the NE-untreated group was subjected to preparation of the cell lysate as it is. In terms of the NE-treated group, 6 hours before the preparation of the cell lysate, the test substance and the negative control substance were prepared to a predetermined concentration and NE was prepared to the concentration of 1×10⁻⁶ M with the adipocyte differentiation medium, followed by addition of the resulting mixture to the cells. Microscopic observation was carried out on the day when the test substance was added and before and after the addition of NE, and, for representative cases, photographs were taken using a Hoffman module lens.

### (2) Brown Adipocyte (hereinafter Referred to as BAT)

Cells seeded on a 24-well plate were cultured in a growth medium for 3 days, and the cells were confirmed to have become confluent. After replacing the medium with a differentiation induction medium, the culture was continued for 2 days, and the negative control substance and the test substance were prepared to a predetermined concentration with a maintenance medium, followed by addition of the prepared medium and 4 days of culture. The treatment with NE was carried out in the same manner as in VAC, using the maintenance medium.
Microscopic observation was carried out before and after the addition of NE, and, for representative cases, photographs were taken using a Hoffman module lens.
The constitutions of the test groups are shown in Table 2 and Table 3 below.

**[Table 2]**

| Three types of adipocytes, VAC, SAC and EAC3 | | | | |
|---|---|---|---|---|
| | Group name | NE added | Final concentration of test substance | Number of samples |
| 1 | Control | - | - | 1 |
| 2 | Continuous addition of sample for 4 days | - | 10 µg/mL | 1 |
| 3 | 6 hours of treatment with sample on last day | - | - | 1 |
| 4 | 6 hours of treatment with NE on last day | + | - | 1 |
| 5 | 6 hours of treatment with NE + sample on + last day | + | 10 µg/mL | 1 |
| 6 | Continuous addition of sample for 4 days 6 → 6 hours of treatment with NE on last day | + | 10 µg/mL | 1 |

**[Table 3]**

| BAT | | | | |
|---|---|---|---|---|
| | Group name | NE added | Final concentration of test substance | Number of samples |
| 1 | Control | - | - | 1 |
| 2 | Continuous addition of sample for 4 days | - | 10 µg/mL | 1 |
| 3 | 1 hour of treatment with sample on last day | - | - | 1 |
| 4 | 1 hour of treatment with NE on last day | + | - | 1 |
| 5 | 1 hour of treatment with NE + sample on + last day | + | 10 µg/mL | 1 |
| 6 | Continuous addition of sample for 4 days 6 → 1 hour of treatment with NE on last day | + | 10 µg/mL | 1 |

### Test Results

### Micrograph for Observation of Cell Morphology (VAC)

### Day 0 after Administration of Test Substance (Day 4 after Seeding)

On day 4 after seeding of visceral adipocytes, 1 mL of the culture medium prepared with the normal medium and the test substance was added, and microscopic observation was carried out, during which photographs of representative cases were taken.
By observation of the entire cells in each well, it was confirmed that lipid droplets have begun to accumulate gradually in the adipocytes.
No difference among the groups was observed, and therefore it was confirmed that the test system was being carried out under the same conditions among the groups.

### Day 2 after Administration of Test Substance (Day 6 after Seeding)

In the control group, adipocytes in which small lipid droplets have accumulated were found, although some mature adipocytes were also found.
In the group wherein 10 µg/mL of the composition of the present invention (Example 3) was added, adipocytes in which small lipid droplets have accumulated were predominant, while enlarged adipocytes were hardly found.
In all the groups, cell damage was not observed.

### Day 4 after Administration of Test Substance (Day 8 after Seeding)

In the control group, most adipocytes have become mature cells, and many enlarged cells were observed.
In the group wherein 10 µg/mL of the composition of the present invention (Example 3) was continuously added, mature adipocytes have increased. In addition, some enlarged adipocytes were found. Compared to the control group, no large difference was observed.
The effect of 6 hours of the treatment with the sample was not clearly observed.
The reaction by 6 hours of the treatment with NE was obtained. Further, the continuous addition of the sample appeared to have increased the reactivity to NE. With 6 hours of treatment with NE + the sample, it appeared that the reactivity was equivalent to that in the group with 6 hours of treatment with NE.

### Day 6 after Administration of Test Substance (Day 10 after Seeding)

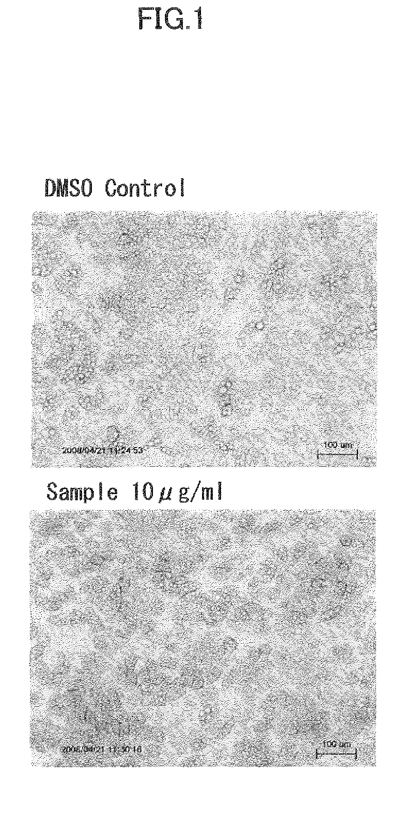

In the control group, there were many enlarged cells, and fusion of lipid droplets was observed.
In the group to which the sample was added, enlarged cells were hardly observed, and therefore the enlargement-suppressing action was clearly confirmed.
Micrographs taken during observation of cell morphology (VAC) (×100 magnification) are shown in Fig. 1.

### Micrographs Taken during Observation of Cell Morphology (SAC)

### Day 0 after Administration of Test Substance (Day 4 after Seeding)

On day 4 after seeding of subcutaneous white adipocytes, 1 mL of the culture medium prepared with the normal medium and the test substance was added, and microscopic observation was carried out, during which photographs of representative cases were taken.
By observation of the entire cells in each well, it was confirmed that lipid droplets have begun to accumulate gradually in the adipocytes.
No difference among the groups was observed, and therefore it was confirmed that the test system was being carried out under the same conditions among the groups.

### Day 2 after Administration of Test Substance (Day 6 after Seeding)

In the control group, adipocytes in which small lipid droplets have accumulated were found, although some mature adipocytes were also found.
In the group wherein 10 µg/mL of the composition of the present invention (Example 3) was added, adipocytes in which small lipid droplets have accumulated were predominant, while enlarged adipocytes were hardly found.
In the group wherein 10 µg/mL of the composition of the present invention (Example 3) was added, a phenomenon assumed to be due to detachment of the cells was slightly observed, wherein decrease in the cell density was confirmed.

### Day 4 after Administration of Test Substance (Day 8 after Seeding)

In the control group, it was confirmed that most adipocytes have become mature cells.
In the group wherein 10 µg/mL of the composition of the present invention (Example 3) was added, mature adipocytes have increased. Further, the phenomenon observed on day 6 wherein detachment of the cells was assumed had proceeded, and the number of adipocytes was smaller than in the control group.
In the group with 6 hours of treatment with the sample, the effect was not observed based on observation of morphology.
The reaction by the addition of NE was obtained. Further, it was suggested that the reaction may have been allowed to proceed well by the simultaneous addition of the sample.

### Micrographs Taken during Observation of Cell Morphology (EAC)

### Day 0 after Administration of Test Substance (Day 4 after Seeding)

On day 4 after seeding of periepididymal adipocytes, 1 mL of the culture medium prepared with the normal medium and the test substance was added, and microscopic observation was carried out, during which photographs of representative cases were taken.
By observation of the entire cells in each well, it was confirmed that lipid droplets have begun to accumulate gradually in the adipocytes.
No difference among the groups was observed, and therefore it was confirmed that the test system was being carried out under the same conditions among the groups.

### Day 2 after Administration of Test Substance (Day 6 after Seeding)

In the control group, adipocytes in which small lipid droplets have accumulated were also found, but it was confirmed that accumulation of fat was proceeding.
In the group wherein 10 µg/mL of the composition of the present invention (Example 3) was added, there were more cells having small lipid droplets compared to the control group, and adipocytes having enlarged lipid droplets were hardly observed.
Since, compared to the control group, the cell density was low, there was a possibility of detachment of the cells.

### Day 4 after Administration of Test Substance (Day 8 after Seeding)

In the control group, it was confirmed that most adipocytes have become mature cells, and many cells having enlarged lipid droplets were observed.
In the group wherein 10 µg/mL of the composition of the present invention (Example 3) was added, some adipocytes having enlarged lipid droplets were observed, but, in comparison with the control group, the number of the adipocytes having enlarged lipid droplets was small. Further, since the cell density was low, there was a possibility of detachment of the cells.
The effect of 6 hours of the treatment with the sample was not clearly observed.
The reaction by the addition of NE was obtained. The reactivity to NE achieved by the simultaneous addition or the continuous addition of the sample was at about the same level as that in the group treated with NE for 6 hours.

### Micrographs Taken during Observation of Cell Morphology (BAT)

### Day 0 after Administration of Test Substance (Day 5 after Seeding)

On day 5 after seeding of brown adipocytes, 1 mL of the culture medium prepared with the normal medium and the test substance was added, and microscopic observation was carried out, during which photographs of representative cases were taken.
By observation of the entire cells in each well, it was confirmed that lipid droplets have begun to accumulate gradually in the adipocytes.
No difference among the groups was observed, and therefore it was confirmed that the test system was being carried out under the same conditions among the groups.

### Day 4 after Administration of Test Substance (Day 9 after Seeding)

In the control group, it was confirmed that most adipocytes have become mature cells.
In the group wherein 10 µg/mL of the composition of the present invention (Example 3) was continuously added, it appeared that the sizes of lipid droplets were small and the cell density was low.
The effect of 1 hour of stimulation by the addition of the sample was not clearly confirmed by observation of morphology.
The reaction by 1 hour of the treatment with NE could be sufficiently confirmed.
Further, also in terms of stimulation by the simultaneous addition of NE and the sample and by the addition of NE after the continuous addition of the sample, the reaction was confirmed by observation of morphology.

### Formulation Example 1 (Tablet)

To 10 g of the composition produced in Example 1, 10 g of malic acid and 10 g of ascorbic acid were added, and the mixture was dissolved into 1000 ml of water, followed by freeze-drying of the resulting solution. The resultant had a property to instantly dissolve into pure water. To 10 g of this freeze-dried product, 20 g of mannitol, 50 g of lactose and 20 g of polydextrose were added, and the resulting mixture was mixed well, followed by adding 2 g of sucrose fatty acid ester thereto as a binder, to prepare tablets.

### Formulation Example 2 (Granule)

In 100 g of dextrin, 1 g of the composition produced in Example 2 was dispersed, and the resulting mixture was mixed with 900 g of dextrin, followed by fluidized bed granulation to prepare granules.

### Formulation Example 3 (Jelly)

To 1 g of the composition produced in Example 3, 10 g of ascorbic acid was added, and the resulting mixture was dispersed and dissolved in 500 g of liquid sugar. To the resulting solution, 0.1 g of a gelling agent, 0.1 g of a lemon flavoring agent and 500 ml of water were added, and the resulting mixture was filled into a plastic container, followed by cooling it to prepare a jelly.

### BRIEF DESCRIPTION OF THE DRAWINGS

Fig. 1 shows micrographs taken during observation of cell morphology (VAC) on day 6 after administration of the test substance (day 10 after seeding) (×100 magnification).

## Claims

1. A human adrenergic ß₃ receptor ligand comprising the three components described below:
(A) an unsaturated fatty acid having not less than three double bonds or a pharmaceutically acceptable salt thereof;
(B) an unsaturated fatty acid having one or two double bond(s) or a pharmaceutically acceptable salt thereof; and
(C) a saturated fatty acid or a pharmaceutically acceptable salt thereof.

2. The human adrenergic ß₃ receptor ligand according to claim 1, wherein the number of carbon atoms in the unsaturated fatty acid of the component (A) is 8 to 24.

3. The human adrenergic ß₃ receptor ligand according to claim 1 or 2, wherein the number of carbon atoms in the unsaturated fatty acid of the component (B) is 8 to 24.

4. The human adrenergic ß₃ receptor ligand according to any one of claims 1 to 3, wherein the number of carbon atoms in the saturated fatty acid of the component (C) is 8 to 24.

5. The human adrenergic ß₃ receptor ligand according to any one of claims 1 to 4, wherein the mass ratio between the component (A) and the component (B) is 1:90 to 8:2.

6. The human adrenergic ß₃ receptor ligand according to any one of claims 1 to 5, wherein the ratio between the total mass of the component (A) and the component (B) and the mass of the component (C) is 50:1 to 1:3.

7. A food product containing the human adrenergic ß₃ receptor ligand according to any one of claims 1 to 6.

8. A pharmaceutical containing the human adrenergic ß₃ receptor ligand according to any one of claims 1 to 6.
